(19) Europäisches Patentamt
European Patent Office
Office européen des brevets

(11) Publication number: **0 446 718 A2**

(12) **EUROPEAN PATENT APPLICATION**

(21) Application number: **91103074.0**

(51) Int. Cl.[5]: **C07D 207/452**, C07D 403/12

(22) Date of filing: **01.03.91**

(30) Priority: **08.03.90 IL 93686**
**20.02.91 IL 97302**

(43) Date of publication of application:
**18.09.91 Bulletin 91/38**

(84) Designated Contracting States:
**BE CH DE ES FR GB IT LI NL**

(71) Applicant: **Bromine Compounds Ltd.**
**Makleff House P.O.B. 180**
**Beer-Sheva 84101(IL)**

(72) Inventor: **Eidelman, Chaim**
**Mitzpa Tal-El**
**Doar Na Ma'aleh HaGalil(IL)**
Inventor: **Shorr, Leonard**
**39, Palmach Street**
**Haifa(IL)**
Inventor: **Hermolin, Joshua**
**14, Ha'Havoda Street**
**Ramat-Hasharon 47445(IL)**
Inventor: **Oren, Jakob**
**8/9, Reuven Street**
**Qiryat Bialik(IL)**
Inventor: **Adda, Michel**
**30, Palmach Street**
**Haifa(IL)**

(74) Representative: **Perani, Aurelio et al**
**c/o JACOBACCI-CASETTA & PERANI S.p.A 7,**
**Via Visconti di Modrone**
**I-20122 Milano(IT)**

(54) **Phenoxy bis-maleimides and process for their preparation.**

(57) Compounds of the formula:

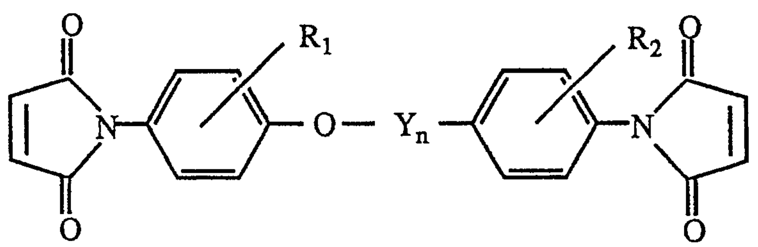

(I)

wherein:

n = 0 or 1;

$R_1$ and $R_2$ are $C_1$ to $C_3$ alkyl groups, or $R_2$ may be H when n = 0; and

Y is selected from the group:

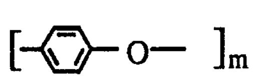 ; 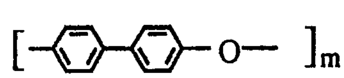

or

EP 0 446 718 A2

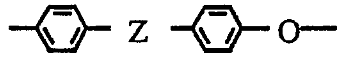

wherein:

m = 1 - 6;

Z = $SO_2$, S, C = O, $CH_2$ or $C(CH_3)_2$;

and processes for their preparation are described.

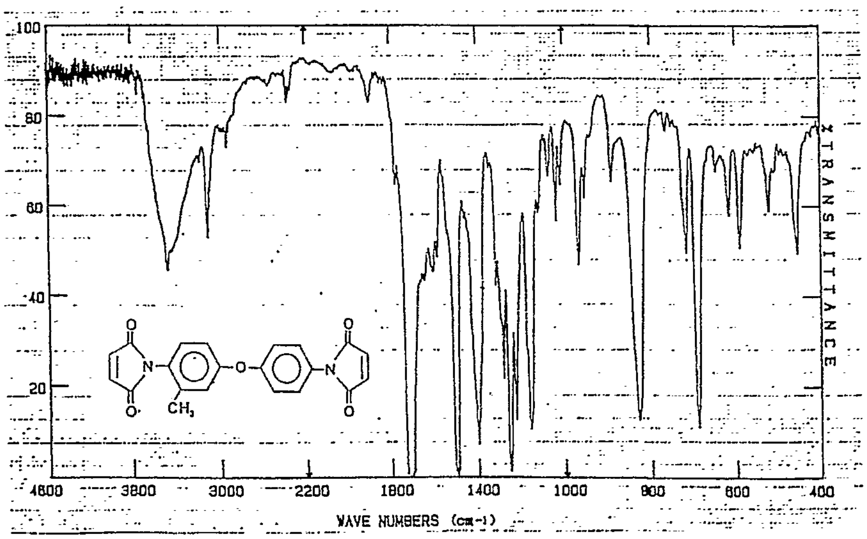


Fig. 6

## Field of The Invention

The present invention relates to novel bis-maleimides and to a process for their preparation.

## SUMMARY OF THE INVENTION

The compounds according to the invention have the formula:

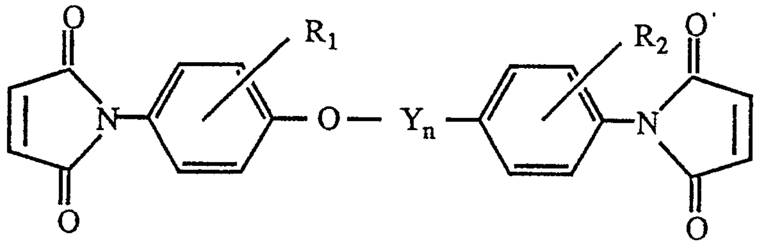

(I)

wherein:

n = 0 or 1;

$R_1$ and $R_2$ are $C_1$ to $C_3$ alkyl groups, or $R_2$ may be H when n=0; and

Y is selected from the group:

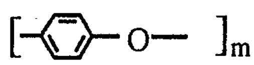
;
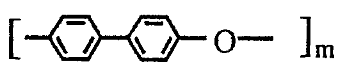

or

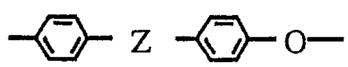

wherein:

m = 1 - 6;

Z = $SO_2$, S, C = O, $CH_2$ or $C(CH_3)_2$.

Compounds in which n = 0 and $R_1$ and $R_2$ are both isopropyl are known in the art and are not claimed herein.

Illustrative, but non-limitative, compounds of formula I include:

4,4'-diamino-3-methyldiphenylether-bis-maleimide,
2,2'-dimethyl-4,4'-diaminotriphenylether-bis-maleimide,
3,3'-dimethyl-4,4'-diaminotriphenylether-bis-maleimide,
2-isopropyl-4,4'-diaminodiphenylether-bis-maleimide,
2,2'-dimethyl-4,4'-diaminotetraphenylether-bis-maleimide,
bis [4-amino-2-methylphenoxy] biphenyl-bis-maleimide,
bis [4-amino-3-methylphenoxy] biphenyl-bis-maleimide,
bis [4-amino-2-methylphenoxy] diphenylsulfone-bis-maleimide,
bis [4-amino-3-methylphenoxy] diphenylsulfone-bis-maleimide,
bis [4-amino-2-methylphenoxy] diphenylsulfide-bis-maleimide,
bis [4-amino-3-methylphenoxy] diphenylsulfide-bis-maleimide,
bis [4-amino-2-methylphenoxy] diphenylether-bis-maleimide,
bis [4-amino-3-methylphenoxy] diphenylether-bis-maleimide,
bis [4-amino-2-methylphenoxy] benzophenone-bis-maleimide,
bis [4-amino-3-methylphenoxy] benzophenone-bis-maleimide,
bis [4-amino-2-methylphenoxy] 2,2-diphenylpropane-bis-maleimide,

bis [4-amino-3-methylphenoxy] 2,2-diphenylpropane-bis-maleimide,
bis [4-amino-2-methylphenoxy] diphenylmethane-bis-maleimide, and
bis [4-amino-3-methylphenoxy] diphenylmethane-bis-maleimide.

Compounds of formula I are prepared by reacting the corresponding diamino compound of formula:

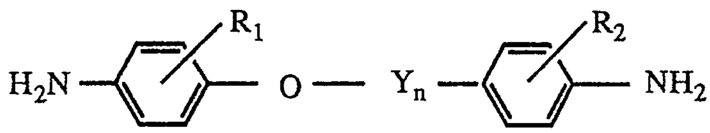

(II)

wherein $R_1$, $R_2$, Y and n have the same meanings as hereinbefore defined with respect to formula I, with maleic anhydride in organic solvents, followed by the dehydrocyclization of the bismaleic acid obtained thereby.

The intermediate bismaleamic acid compounds have the formula:

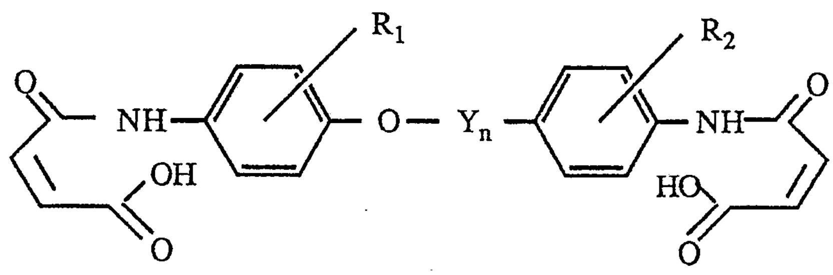

(III)

and are also novel compounds which form part of the present invention.

The preparation of compounds of formula I will be illustrated with particular reference to the preparation of an illustrative compound, 4,4'-diamino-3-methyl-diphenylether-bis-maleimide (DAMDPE-BMI), which will be described in detail hereinafter.

The preparation of other compounds of formula I will be effected in a manner analogous to the preparation of DAMDPE-BMI, as will be further illustrated in the examples.

DAMDPE - BMI is a novel precursor for new polymers. Polymers of this type are well known in the art and have a variety of industrial uses. Among the commercially available bismaleimide resins there are, e.g., Araldite® XU 8292 NPM 60 laminating resin (Ciba-Geigy), which is a polyimide resin useful in the manufacture of high temperature resistant electrical laminates, which is prepared by reacting 4,4'-bis-maleimide diphenylmethane with 0,0'-diallyl Bisphenol A. Another copolymer based on the same monomers, known as Matrimid® 5292 (Ciba-Geigy) is used for high temperature composites and adhesive applications.

4,4'-Disubstituted phenyl ethers of this type are industrially useful monomers for the production of high performance polymers for application in the aviation, electrical, electronics and aerospace industries. Because of the many applications, a wide range of properties are sought by structural changes in the monomers. Thus, altering these monomers by substituents in the aromatic ring, and/or the substitution of one radical which serves to bind two of the aromatic rings by another, affects the softening point, flexibility, heat resistance, etc., of the resins produced therefrom. The improved properties they provide can benefit a wider range of applications which seek better performance from lighter-weight materials. The family of compounds described herein serve to provide this range of variability in the polymer properties.

DAMDPE-BMI has the formula:

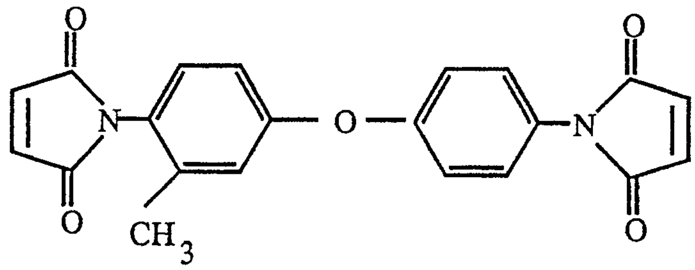

This compound is prepared by reacting 4,4'-diamino-3-methyl-diphenylether (DAMDPE) with maleic anhydride in organic solvents, e.g., following a procedure described by D. Kumar, Chem. & Ind., 21 March, 1981.

The reaction comprises two consecutive steps. In the first step, the bis-amic moiety (4,4'-diamino-3-methyl-diphenylether-bis-maleamic acid = DAMDPE-BMA) is formed, and then the rings are closed by removing water, e.g., by the addition of acetic anhydride. After the completion of water removal the product DAMDPE-BMI can be isolated by crystallization by any of the procedures customary in the art.

The first step of the reaction can be carried out at any temperature which affords high enough reaction rates, without adversely affecting the reactant. Typically, 60° C is a convenient reaction temperature. Thus, in the first step, the intermediate, DAMDPE-BMA, having the formula

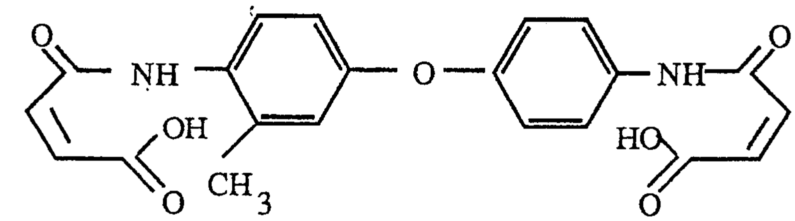

is formed.

Alternative processes for the preparation of the compound of the invention are by a thermal route described, e.g., in Japanese Patent Application No. 159764/82 (Ihara). In this method imidization is achieved by heating DAMDPE-BMA in an inert non-polar solvent, sometimes in the presence of an inert polar aprotic solvent catalyzed by an organic or inorganic acidic compound. The thermal imidization can be carried out continuously or batchwise.

## DETAILED DESCRIPTION OF PREFERRED EMBODIMENTS

### Brief Description of the Drawings

- Fig. 1 is the $^{1}$H-NMR spectrum of 4,4-diamino-3-methyldiphenylether-bis-maleamic acid (DAMDPE-BMA);
- Fig. 2 is the $^{13}$C-NMR spectrum of DAMDPE-BMA;
- Fig. 3 is the IR spectrum of DAMDPE-BMA;
- Fig. 4 is the $^{1}$H-NMR spectrum of DAMDPE-BMI;
- Fig. 5 is the $^{13}$C-NMR spectrum of DAMDPE-BMI;
- Fig. 6 is the IR spectrum of DAMDPE-BMI;
- Fig. 7 is the $^{1}$H-NMR spectrum of 2,2'-dimethyl-4,4'-diaminotriphenylether-BMI (2,2'-DMDATPE-BMI);
- Fig. 8 is the IR spectrum of 2,2'-DMDATPE-BMI;
- Fig. 9 is the $^{13}$C-NMR spectrum of 2,2'-DMDATPE-BMI;
- Fig. 10 is the $^{1}$H-NMR spectrum of 3,3'-dimethyl-4,4'-diaminotriphenylether-BMI (3,3'-DMDATPE-BMI);
- Fig. 11 is the IR spectrum of 3,3'-DMDATPE-BMI;
- Fig. 12 is the $^{13}$C-NMR of 3,3'-DMDATPE-BMI;
- Fig. 13 is the NMR spectrum in $C_6D_6$ of 2-isopropyl-4,4'-diaminodiphenylether-BMI (2-IPADPE-BMI);
- Fig. 14 is the NMR spectrum in DMSO of 2-IPADPE-BMI; and
- Fig. 15 is the IR spectrum of 2-IPADPE-BMI.

The invention will now be illustrated in detail, with reference to the following examples which are provided for the purpose of illustration and which are not intended to constitute a limitation of the invention.

## PREPARATION OF STARTING MATERIALS

### A) Preparation of DBMDPE

The starting material, 4,4'-dibromo-3-methyldiphenylether (DBMDPE) was prepared as follows.

To a four-necked round-bottomed flask equipped with a stirrer, a condenser, a dropping funnel and a thermometer, containing a stirred solution of m-phenoxytoluene (m-PHT) (460 g, 2.5 moles) in one liter of dichloromethane, there were added 840 g ( 5.25 moles) of $Br_2$. The top of the condenser was equipped with a trap to absorb HBr released during the reaction. $Br_2$ was added at a temperature between -5° and 0° C and in complete darkness, during one hour and, after addition, the reaction mixture was stirred for an additional 1 hour at about 25° C. The progress of the reaction was determined by GC. Excess bromine and traces of HBr were neutralized with aqueous 10% $NH_3$ (150 ml). Two phases formed in the reaction, which were separated, and the organic layer was washed with water (200 ml). After distillation of the solvent in the organic phase, crude DBMDPE (820 g) was obtained, containing 97% of the desired isomer (determined by GC) which represents a yield of 93%.

After crystallization from methanol, a product of 99% purity was obtained, containing 46.4% Br, and having a melting point of 44-46° C. The crystallization from ethanol gave identical results.

The product so obtained (DBMDPE) was characterized by GC under the conditions described below, which showed a main peak (99.6%) at a retention time of 7.05 min. The structure of the compound was confirmed by Mass Spectra and NMR, as described in copending Israeli Patent Application No. 93684 of the same applicant herein, the specification of which is incorporated herein by reference.

### B) Preparation of DAMDPE

The starting material 4,4'-diamino-3-methyldiphenylether was prepared as follows: Into a 1 liter 316 Stainless Steel autoclave, there were added DBMDPE (102.6g, 0.3 mole) and 25% aqueous $NH_3$ (500 ml, 6.5 moles) and $CuSO_4 \cdot 5H_2O$ (10 g, 0.04 mole). The autoclave was sealed and heated to 210° C under rapid stirring (1000 rpm). The progress of the reaction and its completion were followed by means of a graph of the internal pressure of the autoclave versus time and, at the end of the reaction, by analysis of the $Br^-$. After four hours, the autoclave was cooled to room temperature, the pressure was released, and the autoclave was opened. The reaction mixture was filtered and washed with aqueous 25% $NH_3$ (200ml) and with water (500 ml).

DAMDPE (58.2 g) was obtained in a purity of 96 - 98% (determined by qualitative G.C.) and a yield of 88%. After recrystallization from ethyl acetate in the presence of active charcoal, a product of 99% purity was obtained with a m.p. of 153 - 155° C. The same result was obtained when recrystallizing from acetonitrile. The structure of DAMDPE was confirmed by Mass Spectra and NMR, as described in copending Israeli Patent Application No. 93685 of the same applicant herein, the specification of which is incorporated herein by reference.

In the analyses of the intermediates and final product, the following equipment was employed:

GC

Gas - chromatograph - Varian 3400

Oven: Initial temperature 100° C, held 1 min., then raised to 250° C at 15°/min.
Injector: 250° C
Detector (transfer line): 300° C
Column: HP-1 (100% methyl polysiloxane), 5 m x 0.53 mm (megabore)
Injection amounts: 1 μl
Flow: 13 ml/min.
Retention Time:
DBMDPE = 7.05 min.
DAMDPE = 8.11 min.

NMR spectra: Bruker WP 200 MHz

| | $^1H$-NMR | $^{13}C$-NMR |
|---|---|---|
| Solvent: | DMSO - $d_6$ | DMSO - $d_6$ |
| Scans: | 50 | 10,000 |
| Reference: | TMS | TMS |

IR: FTIR Nicolet 5 MX
Range: 400-4600 $cm^{-1}$
Scan: 10 (every 1.0 sec.)
Sample: 0.8 mg/80 mg KBr

**Example 1**

Production of DAMDPE - BMI by the chemical route

To a stirred suspension of DAMDPE (21.4 g, 0.1 mole) in acetone (100 ml) under a nitrogen atmosphere, maleic anhydride (MA) (20.6g, 0.21 mole) was added at 20°C. The pale yellow solid of bis-maleamic acid, formed on the addition of maleic anhydride, was vigorously stirred for a further 30 mins. to complete the reaction. To the continuously stirred reaction mixture in acetone there were added acetic anhydride (30 ml) and fused sodium acetate (8.5 g), and the acetone was allowed to reflux. Refluxing and stirring were continued until the solution became clear (2 hours). The clear brownish yellow solution was poured over crushed ice. The yellow solid obtained on maceration was filtered and washed with water. It was then dried and crystallized from the minimum amount of acetonitrile to give the required DAMDPE-BMI (31.8 g, 85%) as yellow crystals.

**Example 2**

Production of 4,4'-diamino-3-methyldiphenylether-bis-maleamic acid (DAMDPE-BMA)

A solution of DAMDPE (21.4 g, 0.1 moles) in DMF (40 ml) was added dropwise to a solution of maleic anhydride (20.6g, 0.21 moles) in toluene (200ml) at 60°C. The suspension that was formed was stirred for 30 mins., under the same conditions, to complete the reaction. The reaction mixture was cooled to room temperature and filtered.

After filtration, washing with petrol ether (50 ml) and drying, DAMDPE-BMA, a precursor for DAMDPE-BMI was obtained (38.9 g), in a yield of 95%.

The structure of the product was determined by $^1H$-NMR (Fig. 1), $^{13}C$-NMR (Fig. 2) and IR (Fig. 3)

**Example 3**

Production of DAMDPE - BMI by the thermal route

DAMDPE-BMA (38.9 g) was prepared as described in Example 2, filtered, washed with petrol ether and dried.

The DAMDPE-BMA obtained above was reacted according to the procedure detailed in Example 1 of Japanese Patent Publication No. 159764/82, to yield 34.8 g of product, DAMDPE-BMI, representing a yield of 93%.

The structure of the product, DAMDPE-BMI, obtained in Example 1 and in this example was determined by $^1H$-NMR (Fig.4), $^{13}C$-NMR (Fig. 5), and IR (Fig. 6).

When the production was effected in continuous mode, comparable results were obtained.

**Example 4**

Preparation of 2,2'-dimethyl-4,4'-diaminotriphenylether-BMI (2,2'-DMDATPE-BMI)

Powdered 2,2'-dimethyl-4,4'-diaminotriphenylether (32.0 g, 0.1 mole) was added over a period of 50 minutes to a vigorously stirred solution of MA (21.5 g, 0.22 moles) in toluene (600 ml) and DMF (43 ml). The temperature during the operation rose from 30° C to 70° C. The resulting slurry of the bismaleamic acid was stirred for an additional 45 minutes at 70° C.

The temperature was raised to reflux and p-toluenesulfonic acid monohydrate (12.4 g) was introduced. The water which formed was removed by azeotropic distillation in a Dean-Stark trap. The end of the reaction (about 9 hours) was determined by the recovery of 4.8 ml of water.

The reaction mixture was cooled and the resulting slurry was filtered. The filter cake was washed with toluene (50 ml) and water (100 ml), and dried. 39.7 G of 2,2'-DMDATPE-BMI was obtained and it was identified by NMR and IR analyses (Figs. 7 and 8, respectively), and $C^{13}$ spectrum (Fig. 9).

**Example 5**

Preparation of 3,3'-dimethyl-4,4'-diaminotriphenylether-BMI (3,3'-DMDATPE-BMI)

Powdered 3,3'-dimethyl-4,4'-diaminotriphenylether (32.0 g, 0.1 mole) was added over a period of 50 minutes to a vigorously stirred solution of MA (21.5 g, 0.22 moles) in acetone (960 ml) and DMF (21 ml). The temperature during the operation rose from 30° C to reflux. The resulting slurry of the bismaleamic acid was stirred for an additional 60 minutes at reflux. The reaction mixture was cooled and the resulting precipitate was filtered, washed with acetone (200 ml) and dried. 51.6 G of bismaleamic acid, a yellow powder, was obtained.

Bismaleamic acid was mixed with xylenes (960 ml) and DMA (22.4 ml). The temperature was raised to 100° C and p-toluenesulfonic acid, PTS, (3.5 g) was introduced. The temperature was raised to reflux and the water which formed was removed by azeotropic distillation in a Dean-Stark trap.

The end of the reaction (about 1.5 hours) was determined by the recovery of 3.9 ml water and by complete dissolution of the reactants in the reaction medium. The reaction mixture was concentrated by distillation of solvents under reduced pressure and 100 ml of methanol was introduced for reslurry. The resulting precipitate was filtered, washed with MeOH (50 ml), and dried. 35.2 G of 3,3'-DMDATPE-BMI was obtained. The product was identified by NMR and IR analyses (Figs. 10 and 11, respectively) and by $C^{13}$ spectrum (Fig. 12).

**Example 6**

Preparation of 2-isopropyl-4,4'-diaminodiphenylether-BMI (2-IPDADPE-BMI)

A solution of 2-isopropyl-4,4'-diaminodiphenylether (24.2 g, 0.1 mole) in acetone (125 ml) was added over a 50 minute period to a vigorously stirred solution of MA (21.5 g, 0.22 mole) in acetone (200 ml) at 50° C. The reaction mixture was stirred for an additional 2 hours at 50° C.

Triethylamine (100 ml), acetic anhydride (25 ml) and $Ni(OAc)_2 \cdot 4H_2O$ (0.08 g) were introduced and the mixture was stirred for an additional 2 hours. At the end of the reaction the mixture was poured into a vigorously stirred mixture of ice and water (about 1 liter). The resulting precipitate was filtered, washed with water and dried. 33.8 G of 2-IPDADPE-BMI was obtained. It was identified by NMR (2 spectra) and IR analyses. Fig. 13 is the NMR spectrum in $C_6D_6$, Fig. 14 is the NMR spectrum in DMSO, and Fig. 15 is the IR spectrum.

**Example 7**

Preparation of 2,2'-dimethyl-4,4'-diaminotetraphenylether-BMI

Powdered 2,2'-dimethyl-4,4'-diaminotetraphenylether (41.2 g, 0.1 mole) was added over a period of 50 minutes to a vigorously stirred solution of MA (21.5 g, 0.22 moles) in xylenes (1000 ml) and DMA (25 ml). The temperature during the operation rose from 30° C to 70° C. The resulting slurry of the bismaleamic acid was stirred for an additional 60 minutes at 70° C.

The temperature was raised to 100° C and PTS (6.5 g) was introduced. The temperature was raised to

reflux and the water which formed was removed by azeotropic distillation in a Dean-Stark trap.

The end of the reaction (about 2.5 hours) was determined by the recovery of 3.9 ml water. The reaction mixture was concentrated by distillation of solvents under reduced pressure and 150 ml of methanol was introduced for reslurry. The resulting precipitate was filtered, washed with MeOH (50 ml), and dried. 41.8 G of 2,2'-DMDATTPE-BMI was obtained. Its elemental analysis showed 71.2% C, 4.8% N and 4.3% H; calc'd: 71.3%, 4.9% and 4.2%, respectively.

**Example 8**

Preparation of bis [4-amino-2-methylphenoxy] biphenyl - BMI

Powdered bis [4-amino-2-methylphenoxy] biphenyl (39.6 g, 0.1 mole) was added over a period of 50 minutes to a vigorously stirred solution of MA (21.5 g, 0.22 moles) in xylenes (1000 ml) and DMA (25 ml). The temperature during the operation rose from 30° C to 70° C. The resulting slurry of the bismaleamic acid was stirred for an additional 60 minutes at 70° C.

The temperature was raised to 100° C and PTS (6.5 g) was introduced. The temperature was raised to reflux and the water which formed was removed by azeotropic distillation in a Dean-Stark trap.

The end of the reaction (about 3.5 hours) was determined by the recovery of 3.9 ml water. The reaction mixture was concentrated by distillation of solvents under reduced pressure and 150 ml of methanol was introduced for reslurry. The resulting precipitate was filtered, washed with MeOH (50 ml), and dried. 45.6 G of bis [4-amino-2-methyl-phenoxy] biphenyl-BMI was obtained. Its elemental analysis showed 73.2% C, 5.1% N and 4.3% H; calc'd: 73.4%, 5.0% and 4.3%, respectively.

**Example 9**

Preparation of bis [4-amino-2-methylphenoxy] diphenylsulfone-BMI

A solution of bis [4-amino-2-methylphenoxy] diphenylsulfone (46.0 g, 0.1 mole) in acetone (240 ml) was added over a 50 minute period to a vigorously stirred solution of MA (21.5 g, 0.22 mole) in acetone (350 ml) at 50° C. The reaction mixture was stirred for an additional 2 hours at 50° C.

Triethylamine (190 ml), acetic anhydride (25 ml), and $Ni(OAc)_2 \cdot 4H_2O$ (0.08 g) were introduced and the mixture was stirred for an additional 2 hours. At the end of the reaction the mixture was poured into a vigorously stirred mixture of ice and water (about 2 liters). The resulting precipitate was filtered, washed with water and dried. 56.4 G of bis [4-amino-2-methylphenoxy] diphenylsulfone-BMI was obtained. The formation of the malemide groups was indicated by NMR (disappearance of ab signal of double bond of maleamic acid) and IR (characteristic absorbence of maleimide carbonyls 1710 $cm^{-1}$) analyses. Its elemental analysis showed 66.0% C, 4,4% N, 3.6% H and 5.2% S; calc'd: 65.8%, 4.5%, 3.8% and 5.2%, respectively.

**Examples 10-12**

The BMI derivatives of bis [4-amino-2-methylphenoxy] - 2,2-diphenylpropane, bis [4-amino-2-methylphenoxy] diphenylmethane and bis [4-amino-2-methylphenoxy] benzophenone were prepared following the thermal cyclization procedure of examples 7 and 8. The formation of the maleimide groups was indicated by NMR (disappearance of ab signal of double bond of maleamic acid) and IR (characteristic absorbence of maleimide carbonyls 1710 $cm^{-1}$) analyses.

**Claims**

**1.** A compound of the formula

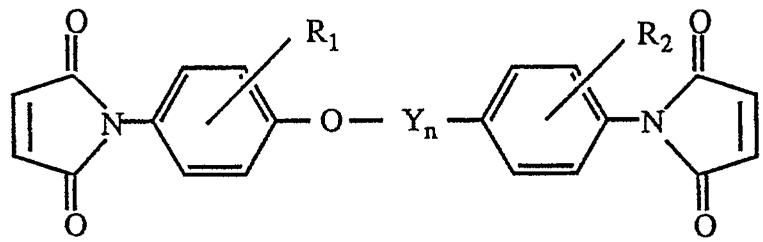

(I)

wherein:

n = 0 or 1;

$R_1$ and $R_2$ are $C_1$ to $C_3$ alkyl groups, or $R_2$ may be H when n = 0; and

Y is selected from the group:

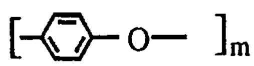

;

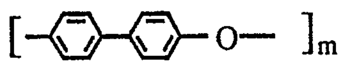

or

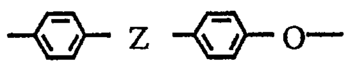

wherein:

m = 1 - 6;

Z = $SO_2$, S, C = O, $CH_2$ or $C(CH_3)_2$;

with the proviso that $R_1$ and $R_2$ cannot both be isopropyl when n = 0.

**2.** 4,4'-Diamino-3-methyldiphenylether-bis-maleimide.

**3.** 2,2'-Dimethyl-4,4'-diaminotriphenylether-bis-maleimide.

**4.** 3,3'-Dimethyl-4,4'-diaminotriphenylether-bis-maleimide.

**5.** 2-Isopropyl-4,4'-diaminodiphenylether-bis-maleimide.

**6.** 2,2'-Dimethyl-4,4'-diaminotetraphenylether-bis-maleimide.

**7.** Bis [4-amino-2-methylphenoxy] biphenyl-bis-maleimide.

**8.** Bis [4-amino-2-methylphenoxy] diphenylsulfone-bis-maleimide.

**9.** Bis [4-amino-2-methylphenoxy] benzophenone-bis-maleimide.

**10.** Bis [4-amino-2-methylphenoxy] 2,2-diphenylpropane-bis-maleimide.

**11.** Bis [4-amino-2-methylphenoxy] diphenylmethane-bis-maleimide.

**12.** A process for the preparation of a compound of formula I, as claimed in claim 1, comprising dehydrocyclizing a compound of the formula

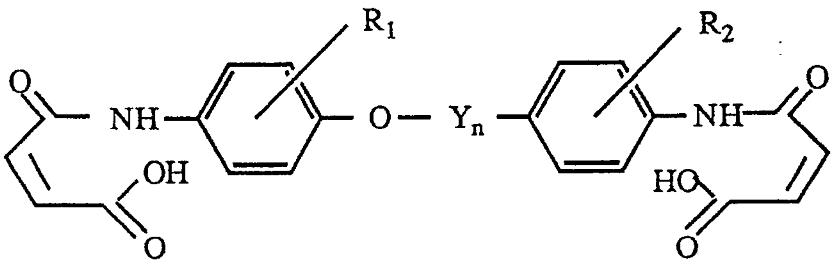 (III)

in an organic solvent.

**13.** A process according to claim 12, wherein dehydrocyclization is effected by chemical dehydrocyclization.

**14.** A process according to claim 12, wherein dehydrocyclization is effected by thermal dehydrocyclization.

**15.** A process according to claim 14, wherein dehydrocyclization is performed in a batch mode.

**16.** A process according to claim 14, wherein dehydrocyclization is performed in continuous mode.

**17.** A compound of formula III, as claimed in claim 12.

**18.** The compound 4,4'-diamino-3-methyl-diphenylether-bis-maleamic acid of formula:

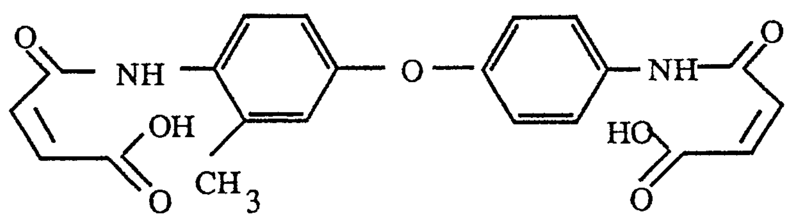

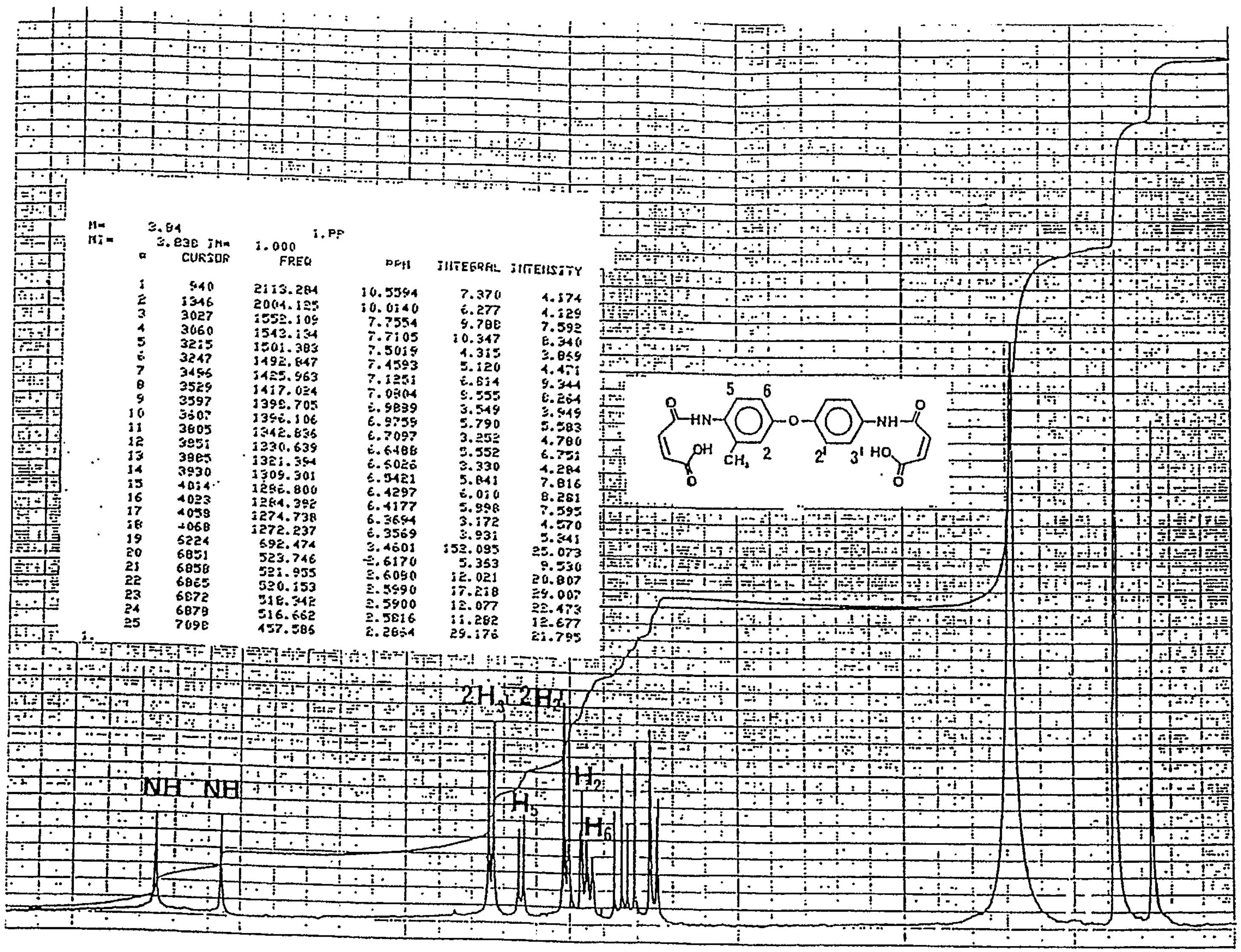

M= 3.84
MI= 3.838 IM= 1.000 1.PP
# CURSOR FREQ PPM INTEGRAL INTENSITY
1 940 2113.284 10.5594 7.370 4.174
2 1346 2004.125 10.0140 6.277 4.129
3 3027 1552.109 7.7554 9.788 7.592
4 3060 1543.134 7.7105 10.347 8.340
5 3215 1501.383 7.5019 4.315 3.869
6 3247 1492.847 7.4593 5.120 4.471
7 3496 1425.963 7.1251 6.814 9.344
8 3529 1417.024 7.0804 9.555 8.264
9 3597 1398.705 6.9839 3.549 3.949
10 3607 1396.106 6.9759 5.790 5.583
11 3805 1342.836 6.7097 3.252 4.780
12 3851 1330.639 6.6488 3.552 6.751
13 3885 1321.394 6.6026 3.330 4.284
14 3930 1309.301 6.5421 5.841 7.816
15 4014 1296.800 6.4297 6.010 8.281
16 4023 1284.392 6.4177 5.998 7.595
17 4059 1274.738 6.3694 3.172 4.570
18 4068 1272.237 6.3569 3.931 5.341
19 6224 692.474 3.4601 152.095 25.073
20 6851 523.746 2.6170 5.353 9.530
21 6858 521.955 2.6090 12.021 20.807
22 6865 520.153 2.5990 17.218 29.007
23 6872 518.342 2.5900 12.077 22.473
24 6879 516.662 2.5816 11.282 12.677
25 7098 457.586 2.2654 29.176 21.795
NH NH
$2H_3$ $2H_2$
$H_5$
$H_2$
$H_6$


Fig. 1

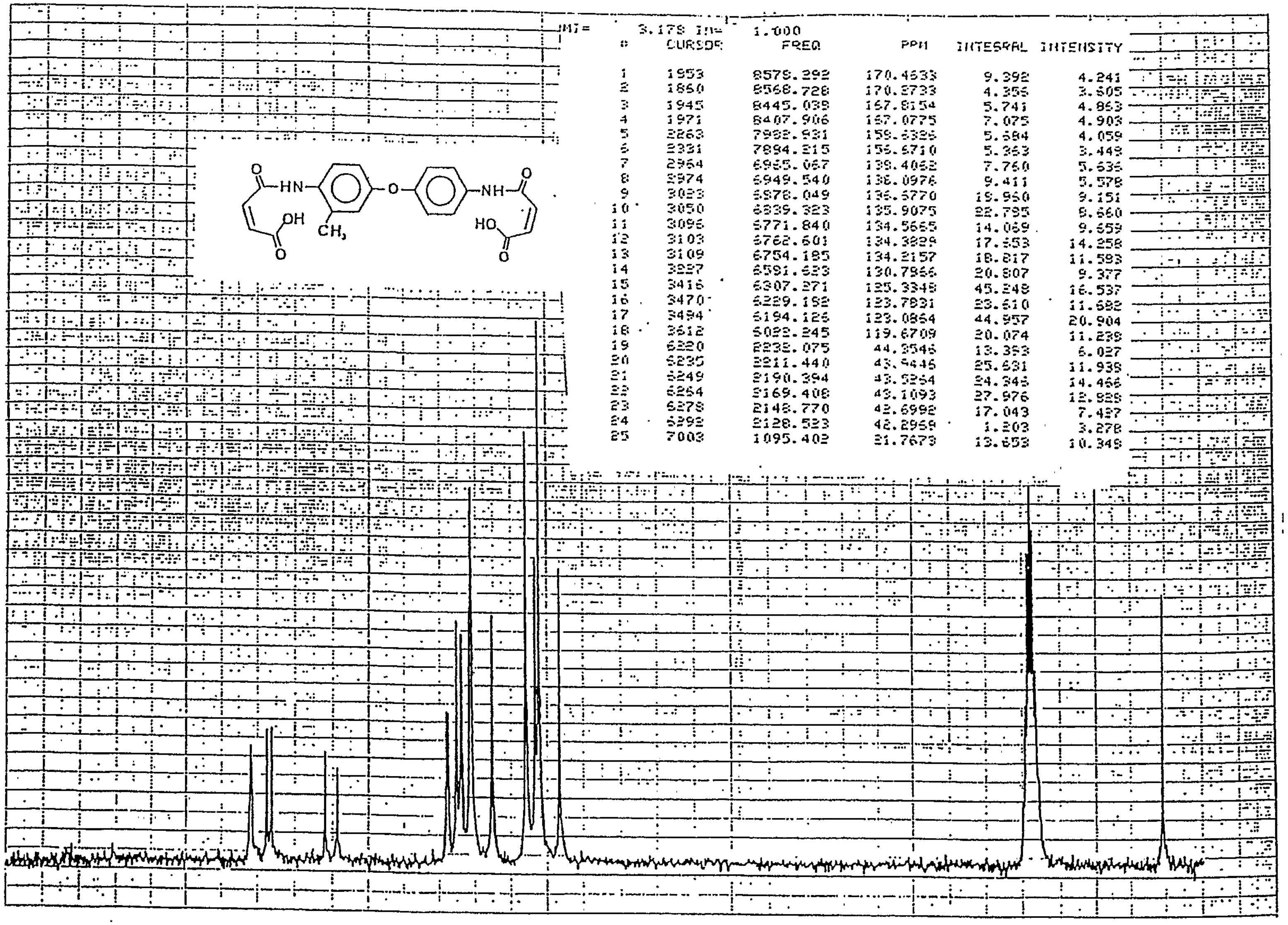


| # | CURSOR | FREQ | PPM | INTEGRAL | INTENSITY |
|---|---|---|---|---|---|
| 1 | 1953 | 8578.292 | 170.4633 | 9.392 | 4.241 |
| 2 | 1860 | 8568.728 | 170.2733 | 4.355 | 3.505 |
| 3 | 1945 | 8445.039 | 167.8154 | 5.741 | 4.863 |
| 4 | 1971 | 8407.906 | 167.0775 | 7.075 | 4.903 |
| 5 | 2263 | 7982.931 | 159.6326 | 5.684 | 4.059 |
| 6 | 2331 | 7894.215 | 156.6710 | 5.363 | 3.449 |
| 7 | 2964 | 6965.067 | 139.4062 | 7.760 | 5.636 |
| 8 | 2974 | 6949.540 | 138.0976 | 9.411 | 5.578 |
| 9 | 3023 | 6878.049 | 136.5770 | 19.960 | 9.151 |
| 10 | 3050 | 6839.323 | 135.9075 | 22.795 | 9.660 |
| 11 | 3096 | 6771.840 | 134.5665 | 14.069 | 9.659 |
| 12 | 3103 | 6762.601 | 134.3829 | 17.653 | 14.258 |
| 13 | 3109 | 6754.185 | 134.2157 | 18.817 | 11.593 |
| 14 | 3227 | 6591.623 | 130.7966 | 20.807 | 9.377 |
| 15 | 3416 | 6307.271 | 125.3348 | 45.248 | 16.537 |
| 16 | 3470 | 6229.192 | 123.7831 | 23.610 | 11.682 |
| 17 | 3494 | 6194.126 | 123.0864 | 44.957 | 20.904 |
| 18 | 3612 | 6022.245 | 119.6709 | 20.074 | 11.239 |
| 19 | 6220 | 2232.075 | 44.3546 | 13.393 | 6.027 |
| 20 | 6235 | 2211.440 | 43.9446 | 25.631 | 11.939 |
| 21 | 6249 | 2190.394 | 43.5254 | 24.345 | 14.466 |
| 22 | 6264 | 2169.408 | 43.1093 | 27.976 | 12.929 |
| 23 | 6278 | 2148.770 | 42.6992 | 17.043 | 7.427 |
| 24 | 6292 | 2128.523 | 42.2969 | 1.203 | 3.278 |
| 25 | 7003 | 1095.402 | 21.7673 | 13.653 | 10.349 |

Fig. 2

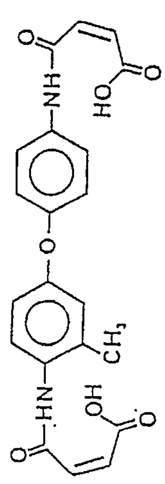

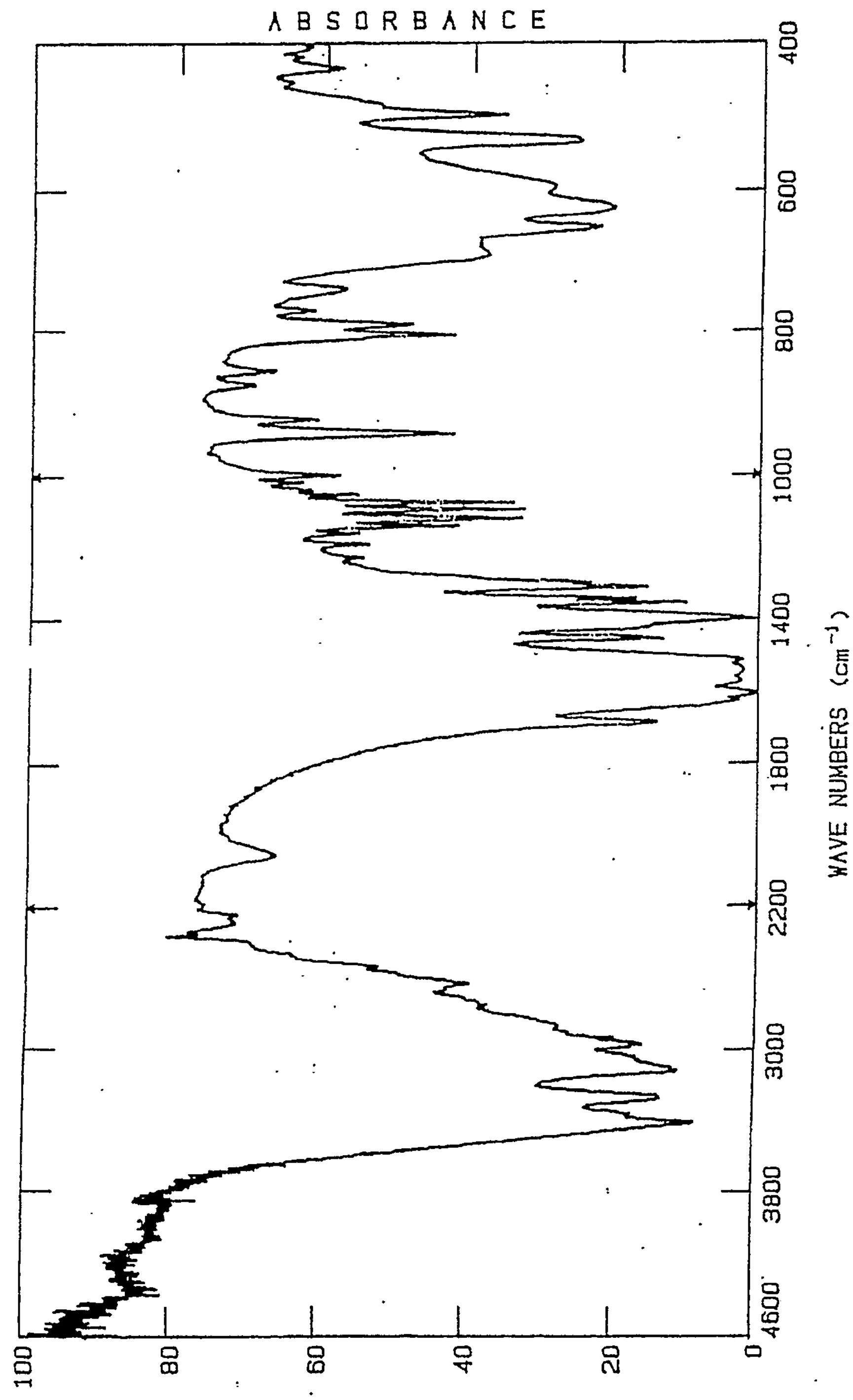
ABSORBANCE
WAVE NUMBERS (cm$^{-1}$)
100
80
60
40
20
0
4600
3800
3000
2200
1800
1400
1000
800
600
400

Fig. 3

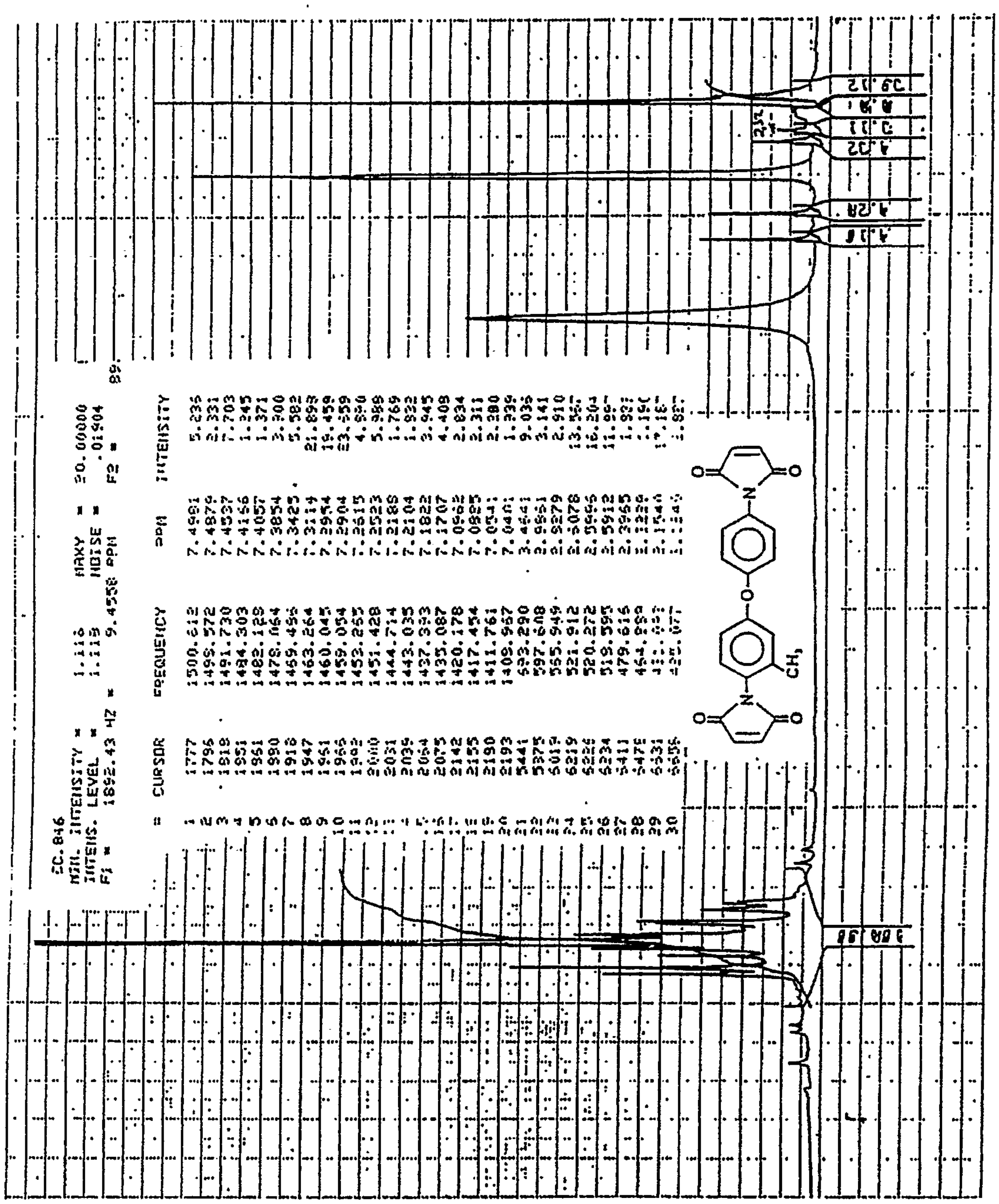
EC.846
MIN. INTENSITY = 1.116 MAXY = 20.00000
INTENS. LEVEL = 1.119 NOISE = .01904
F1 = 1892.43 HZ = 9.4558 PPM F2 =
# CURSOR FREQUENCY PPM INTENSITY
CH3

Fig. 4

| n | CURSOR | FREQUENCY | PPM | INTENSITY |
|---|---|---|---|---|
| 1 | 2284 | 8747.139 | 173.8186 | 6.698 |
| 2 | 2722 | 8078.798 | 160.5375 | 3.132 |
| 3 | 2760 | 8020.956 | 159.3992 | 3.094 |
| 4 | 3315 | 7174.244 | 142.5628 | 3.995 |
| 5 | 3442 | 6980.350 | 139.7099 | 11.461 |
| 6 | 3449 | 6970.258 | 138.5093 | 11.363 |
| 7 | 3574 | 6778.999 | 134.7067 | 5.071 |
| 8 | 3647 | 6667.450 | 132.4921 | 9.362 |
| 9 | 3696 | 6592.593 | 131.0046 | 2.845 |
| 10 | 3734 | 6534.451 | 129.8492 | 2.467 |
| 11 | 3920 | 6250.881 | 124.2142 | 5.189 |
| 12 | 3959 | 6191.319 | 123.0306 | 10.325 |
| 13 | 4044 | 6062.189 | 120.4647 | 5.269 |

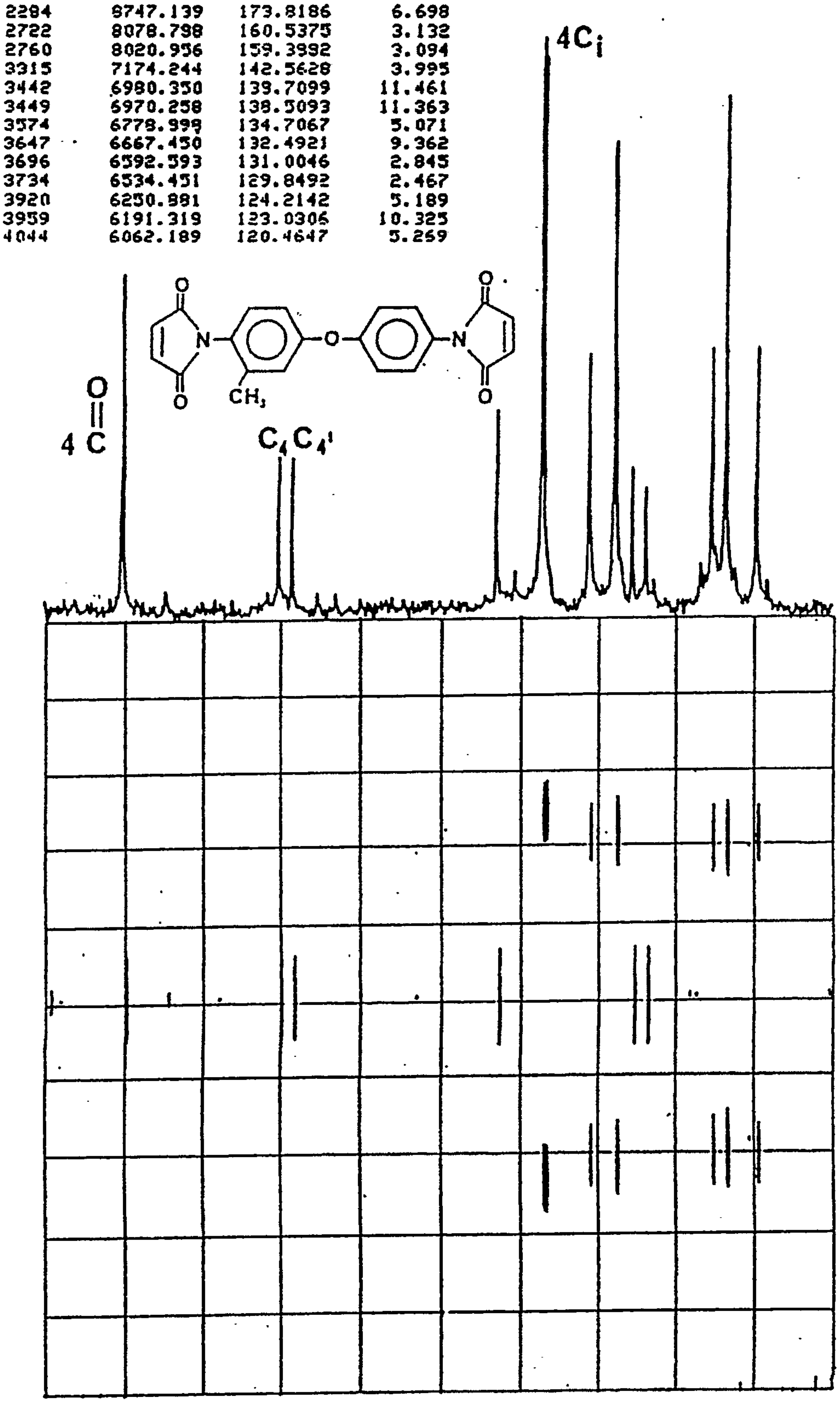


**Fig. 5**

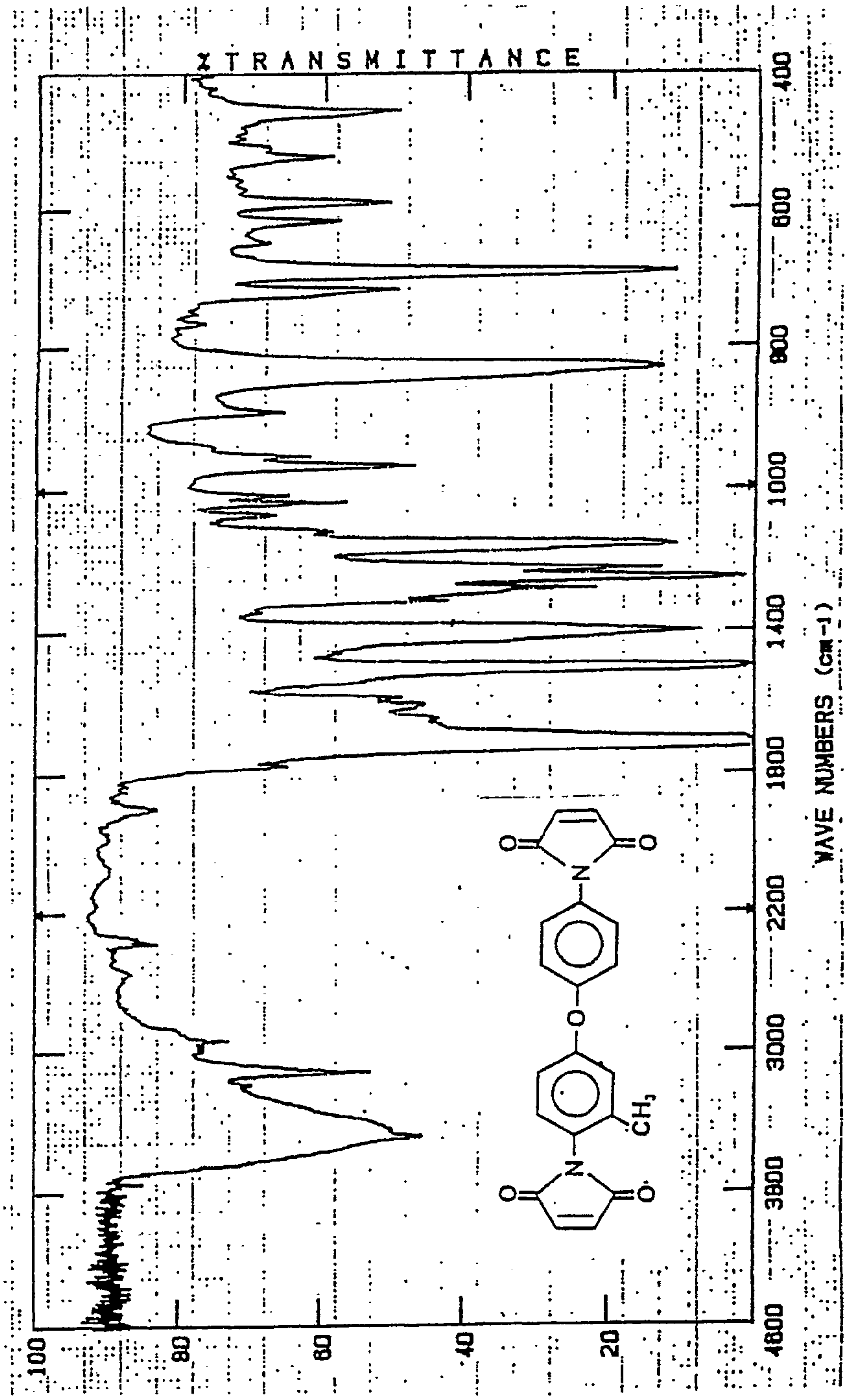
% TRANSMITTANCE
100
80
60
40
20
4000
3600
3000
2200
1800
1400
1000
800
600
400
WAVE NUMBERS (cm-1)

**Fig. 6**

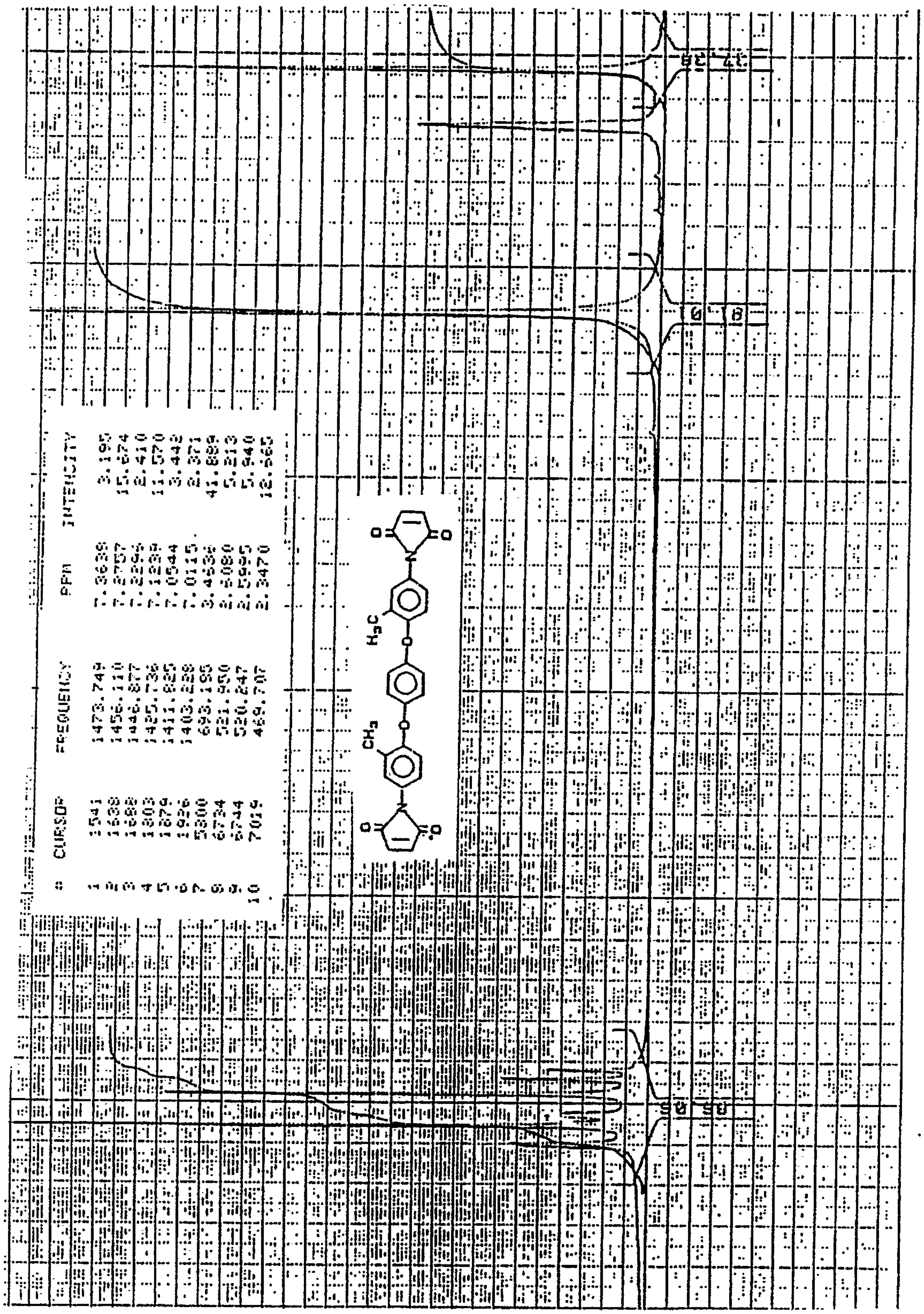

| # | CURSOR | FREQUENCY | PPM | INTENSITY |
|---|---|---|---|---|
| 1 | 1541 | 1473.749 | 7.3639 | 3.195 |
| 2 | 1638 | 1456.110 | 7.2757 | 15.674 |
| 3 | 1688 | 1446.877 | 7.2296 | 2.410 |
| 4 | 1803 | 1425.736 | 7.1239 | 11.570 |
| 5 | 1879 | 1411.825 | 7.0544 | 3.442 |
| 6 | 1926 | 1403.228 | 7.0115 | 2.371 |
| 7 | 5300 | 693.195 | 3.4636 | 41.889 |
| 8 | 6734 | 521.950 | 2.6080 | 5.313 |
| 9 | 6744 | 520.247 | 2.5995 | 5.940 |
| 10 | 7019 | 469.707 | 2.3470 | 12.565 |


Fig. 7

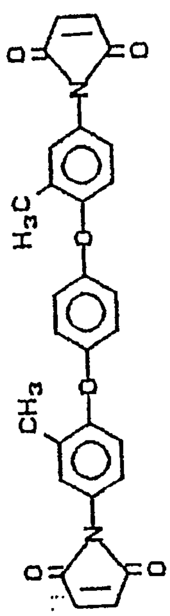

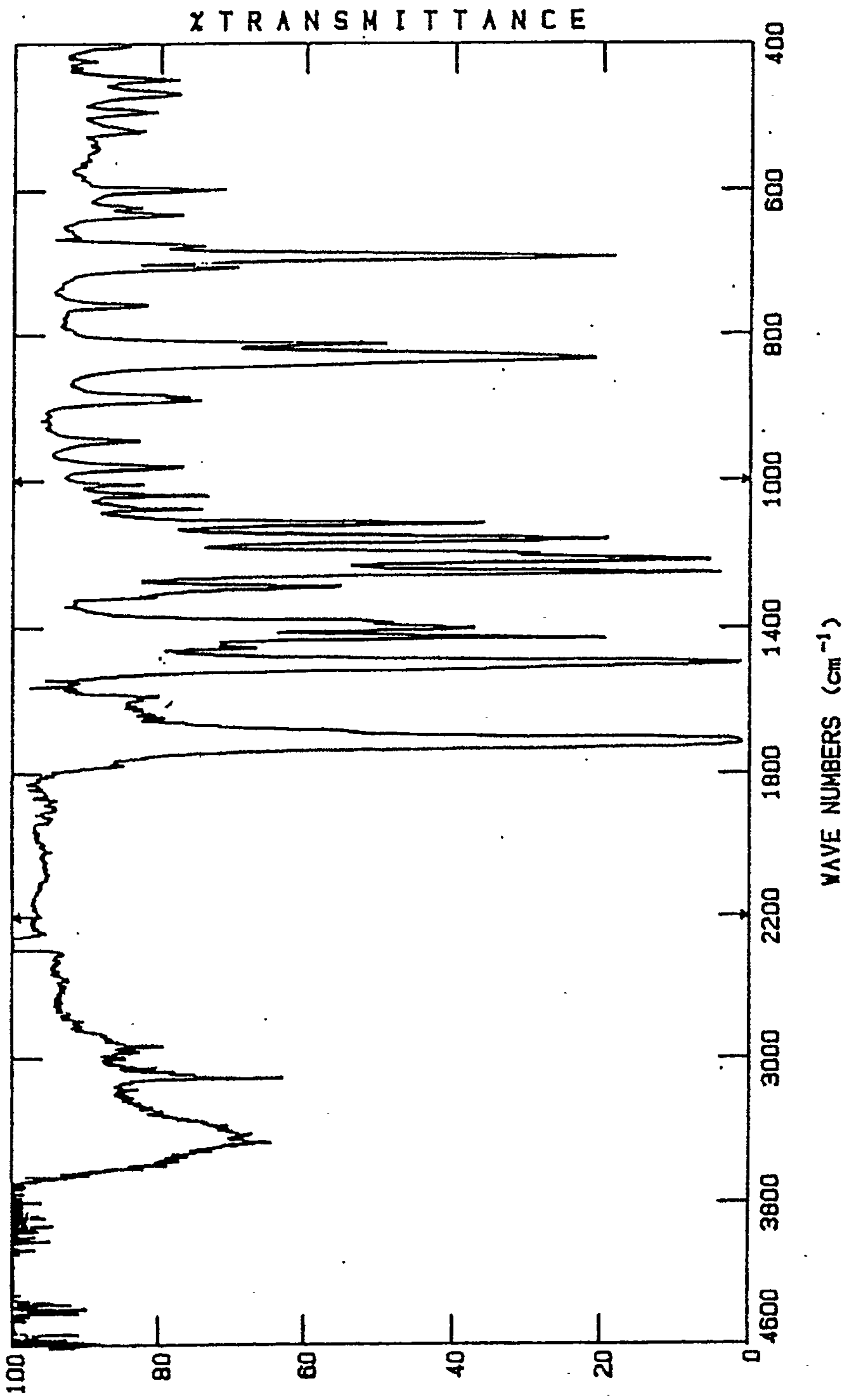
%TRANSMITTANCE
WAVE NUMBERS (cm$^{-1}$)
400
600
800
1000
1400
1800
2200
3000
3800
4600
0
20
40
60
80
100

Fig. 8

Fig. 9

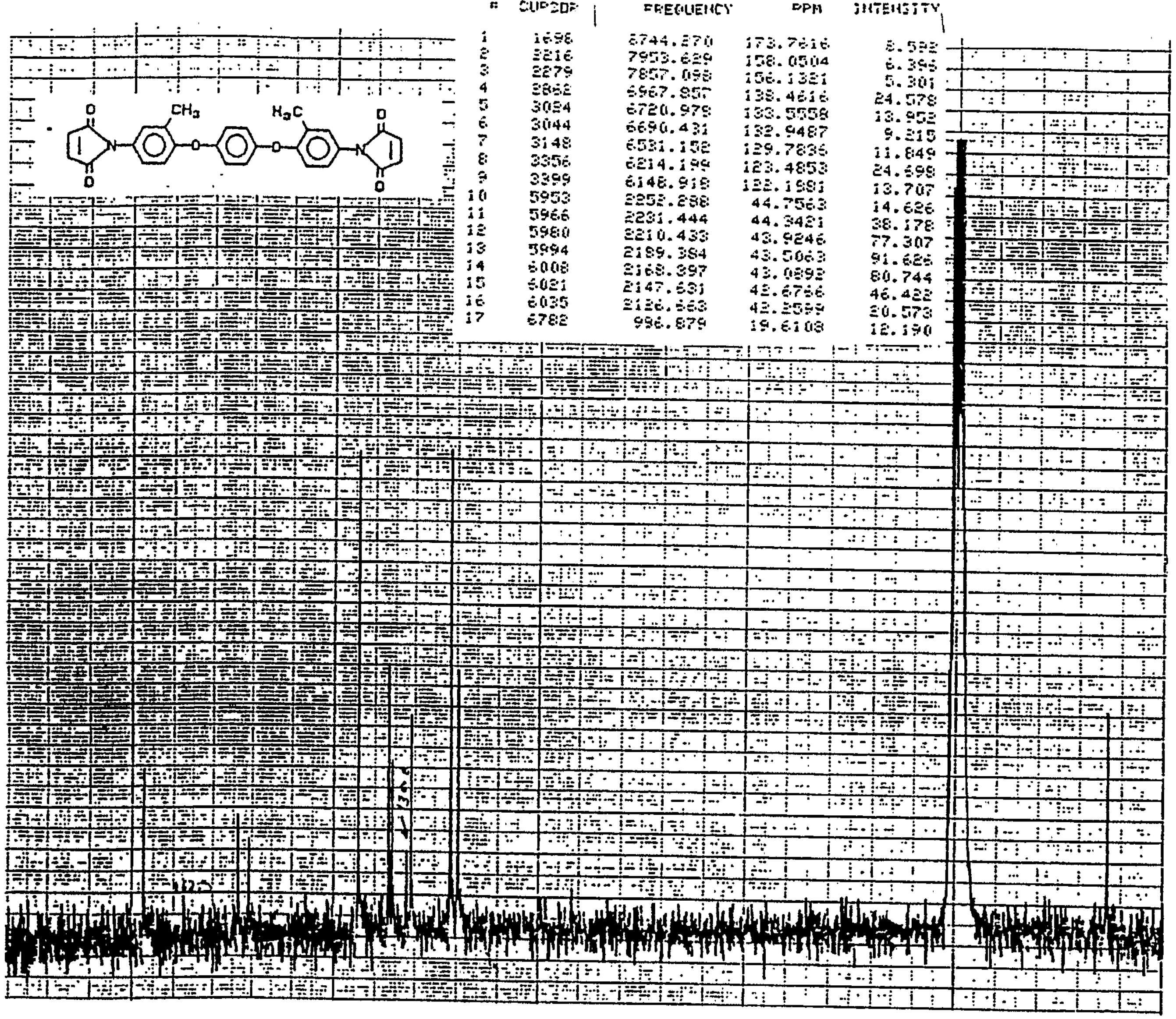

| # | CURSOR | FREQUENCY | PPM | INTENSITY |
|---|---|---|---|---|
| 1 | 1696 | 8744.270 | 173.7616 | 8.592 |
| 2 | 2216 | 7953.629 | 158.0504 | 6.395 |
| 3 | 2279 | 7857.098 | 156.1321 | 5.301 |
| 4 | 2862 | 6967.857 | 138.4616 | 24.578 |
| 5 | 3024 | 6720.979 | 133.5558 | 13.952 |
| 6 | 3044 | 6690.431 | 132.9487 | 9.215 |
| 7 | 3148 | 6531.152 | 129.7836 | 11.849 |
| 8 | 3356 | 6214.199 | 123.4853 | 24.698 |
| 9 | 3399 | 6148.918 | 122.1981 | 13.707 |
| 10 | 5953 | 2252.288 | 44.7563 | 14.626 |
| 11 | 5966 | 2231.444 | 44.3421 | 38.178 |
| 12 | 5980 | 2210.433 | 43.9246 | 77.307 |
| 13 | 5994 | 2189.384 | 43.5063 | 91.626 |
| 14 | 6008 | 2168.397 | 43.0892 | 80.744 |
| 15 | 6021 | 2147.631 | 42.6766 | 46.422 |
| 16 | 6035 | 2126.663 | 42.2599 | 20.573 |
| 17 | 6782 | 996.879 | 19.6108 | 12.190 |

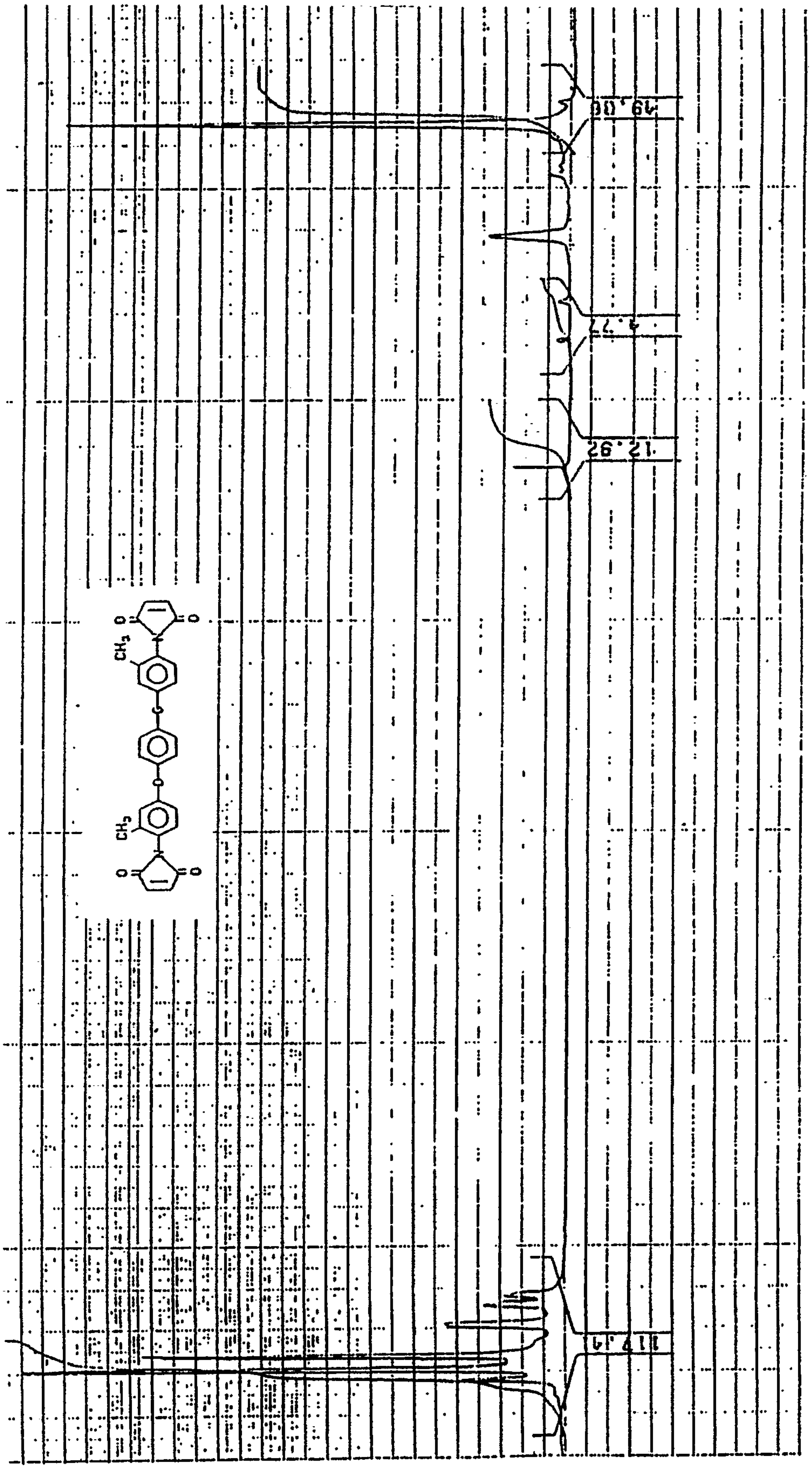

Fig. 10

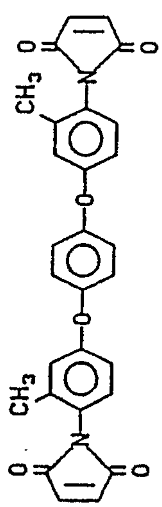

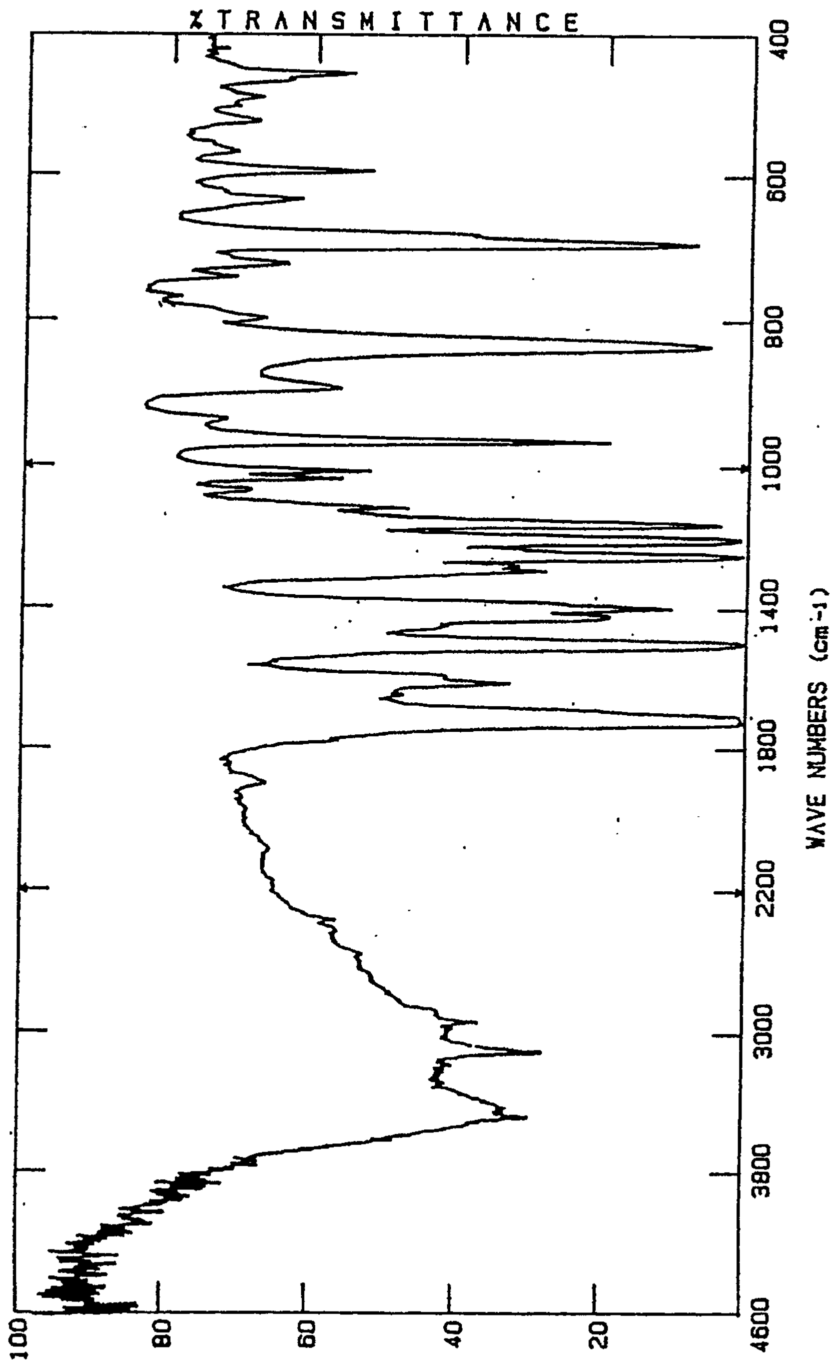
%TRANSMITTANCE
WAVE NUMBERS (cm-1)
100
80
60
40
20
4600
3800
3000
2200
1800
1400
1000
800
600
400

Fig. 11

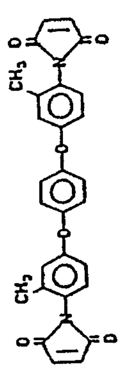

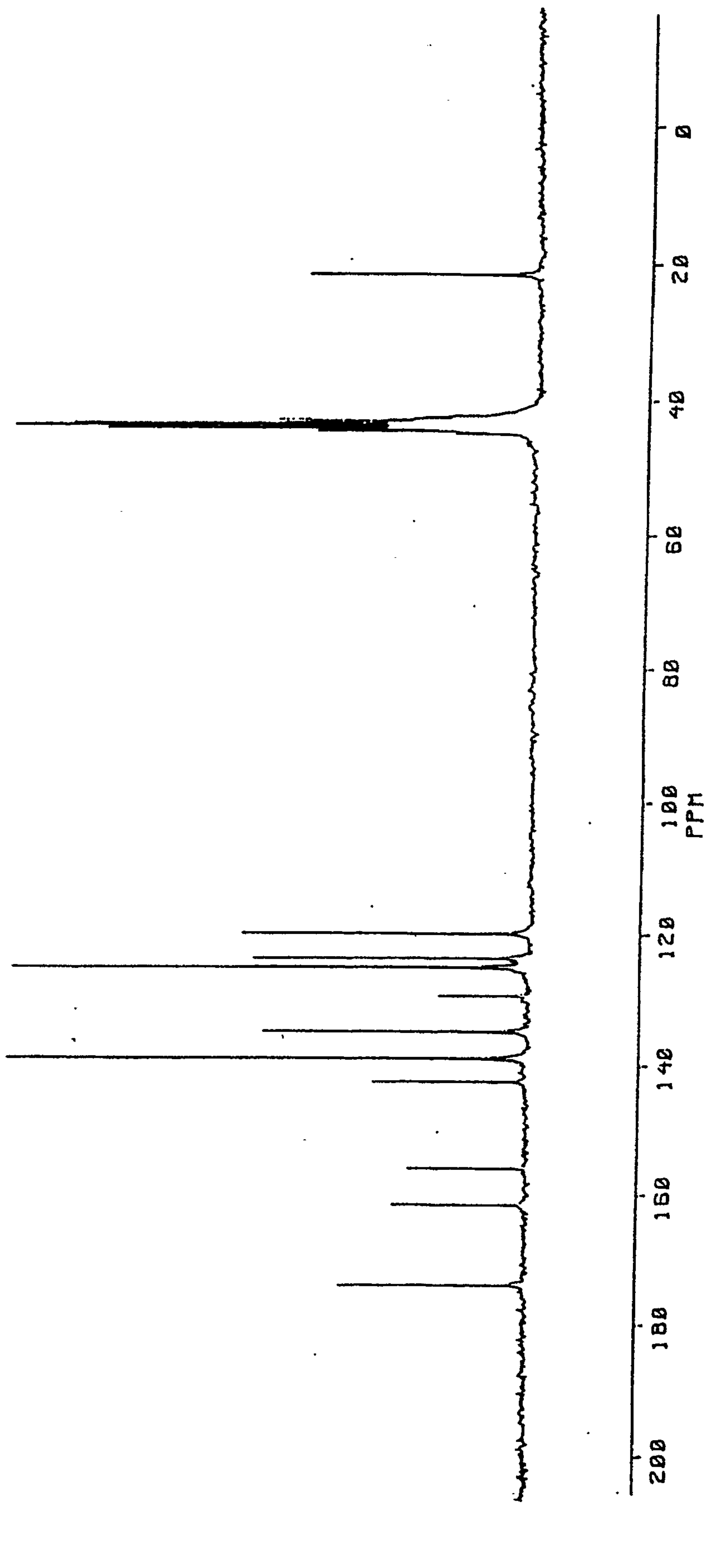


Fig. 12

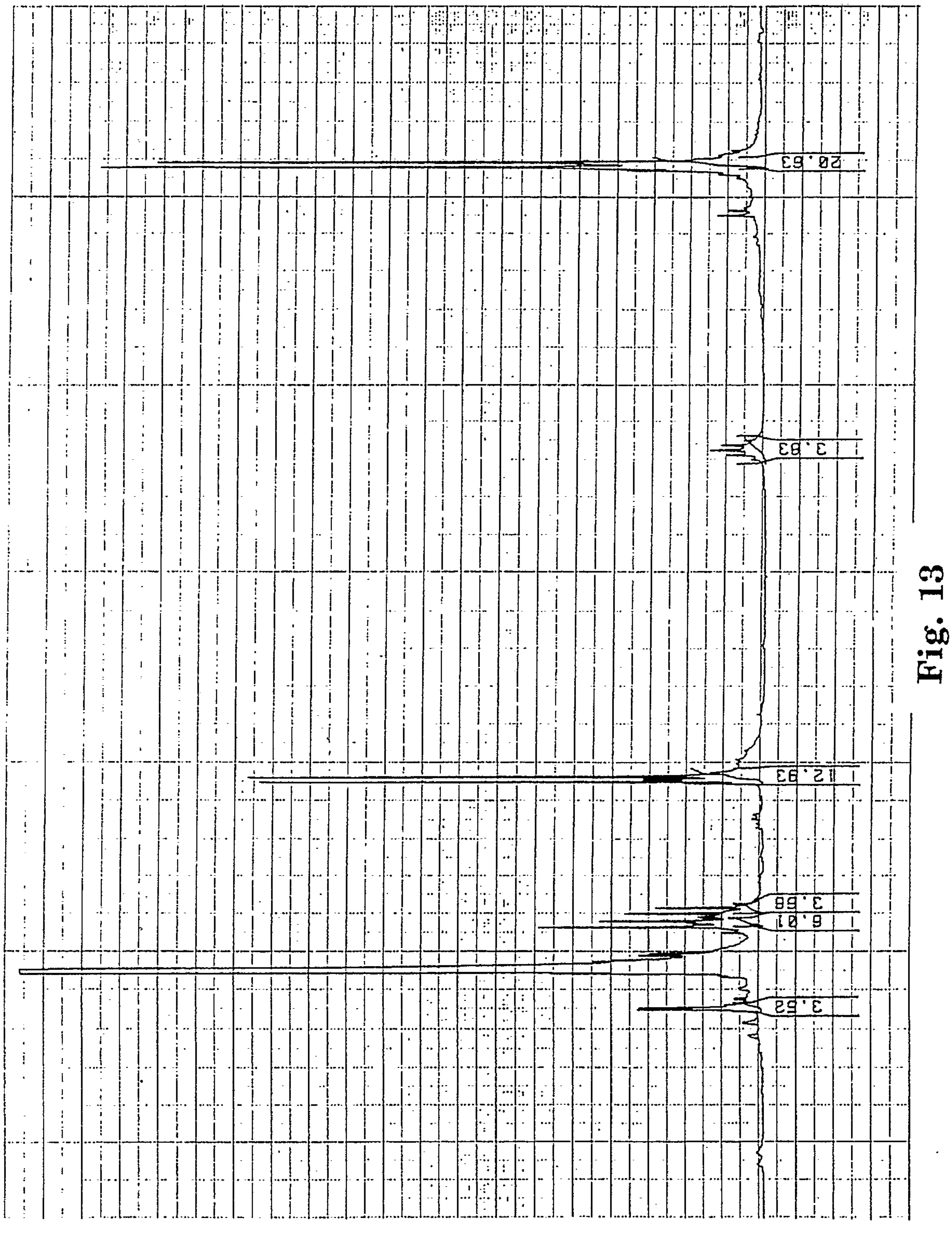


Fig. 13

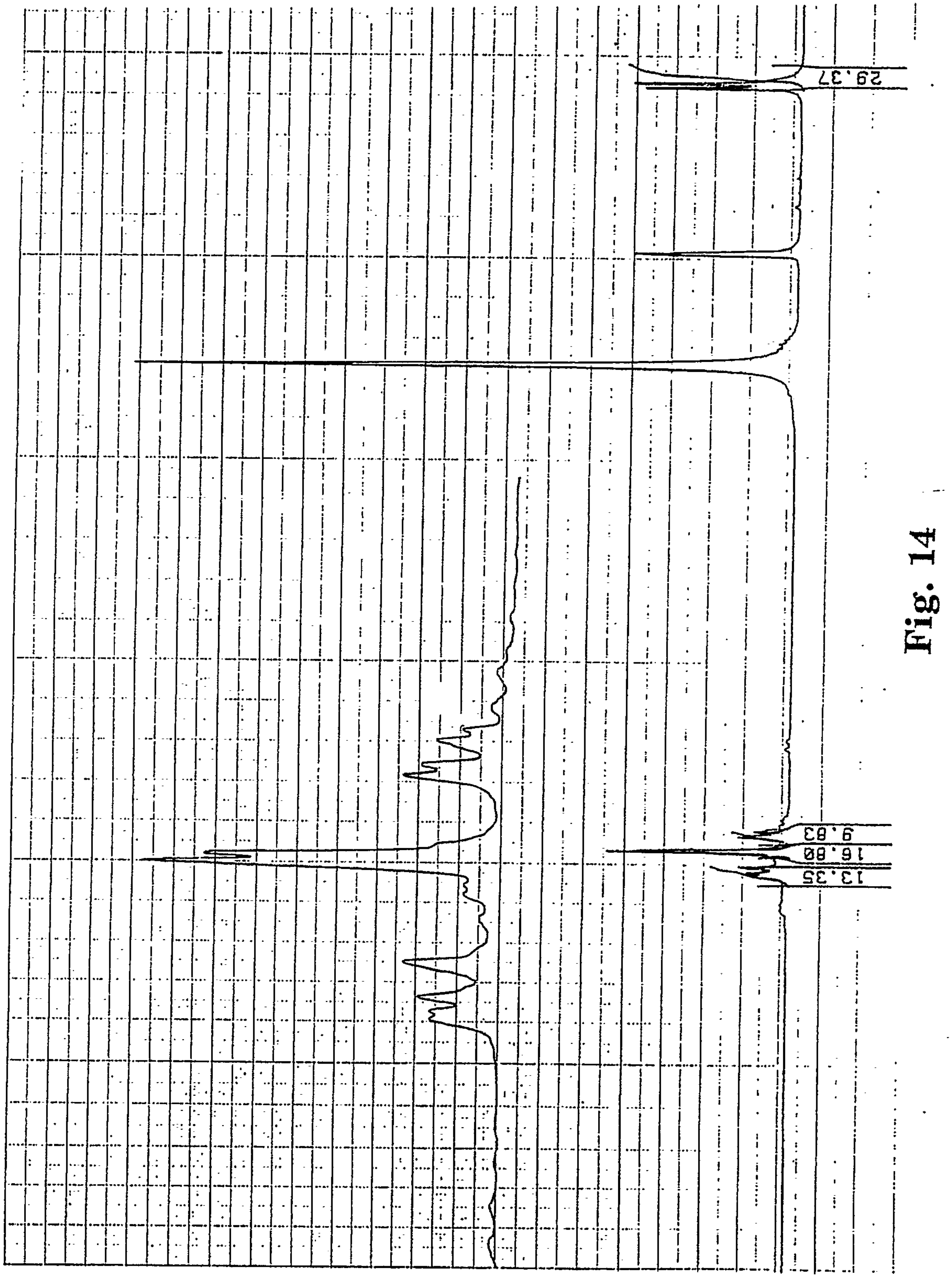


Fig. 14

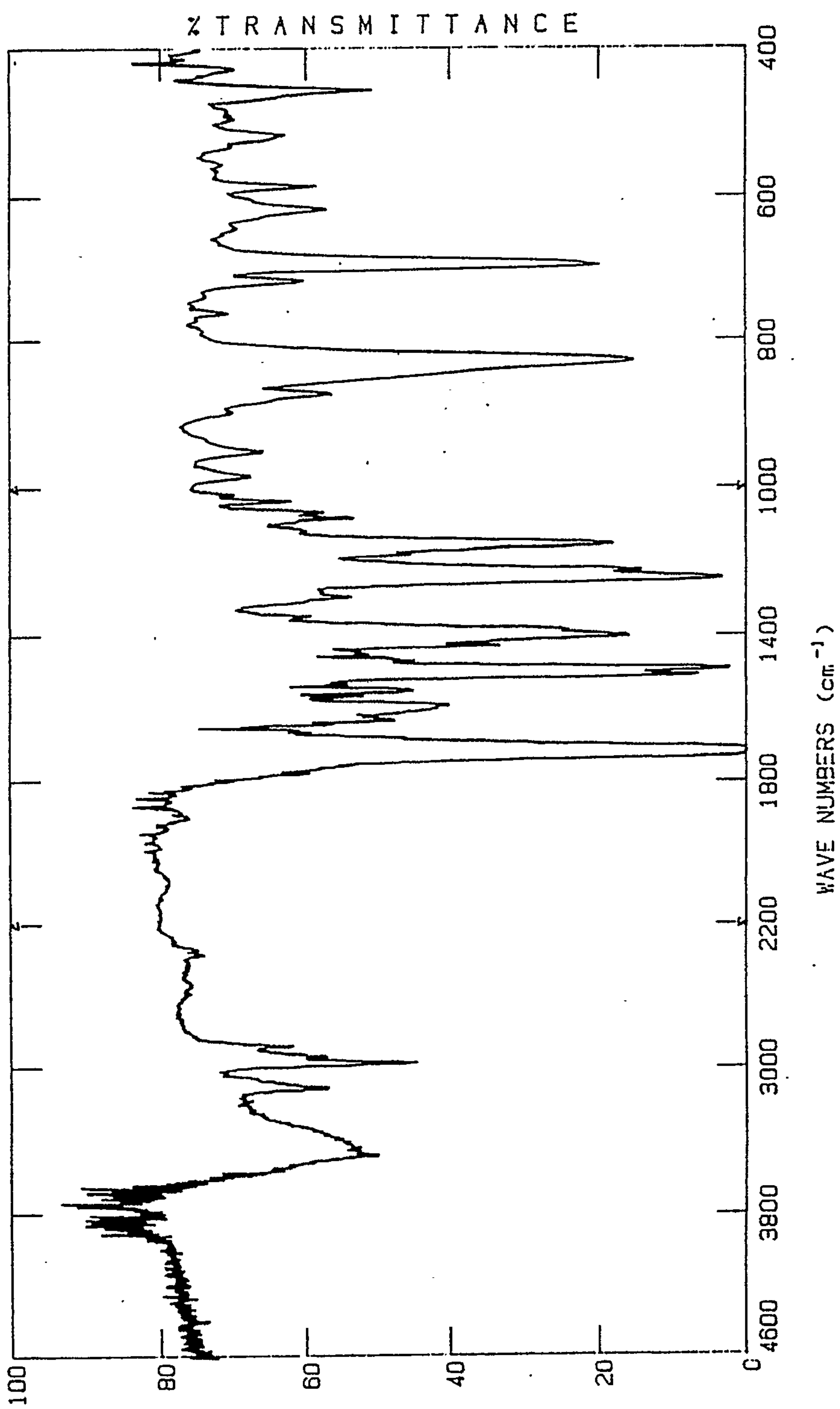
%TRANSMITTANCE
WAVE NUMBERS (cm-1)
100
80
60
40
20
0
4600
3800
3000
2200
1800
1400
1000
800
600
400

Fig. 15